# EUROPEAN PATENT SPECIFICATION

(11) **EP 4 024 113 B1**
(45) Date of publication and mention of the grant of the patent: **25.06.2025**
(21) Application number: 20858181.9
(22) Date of filing: 24.08.2020
(51) Int. Cl.: G02B 23/24, G02B 23/26, A61B 1/00, A61B 1/06

(54) **ENDOSCOPE SYSTEM AND METHOD FOR OPERATING SAME**
ENDOSKOPISCHES SYSTEM UND VERFAHREN ZUM BETRIEB DAVON
SYSTÈME D'ENDOSCOPE ET SON PROCÉDÉ DE FONCTIONNEMENT

(30) Priority: 28.08.2019 JP 2019155553
(43) Date of publication of application: 06.07.2022
(73) Proprietor: FUJIFILM Corporation, Tokyo 106-8620 (JP)
(72) Inventor: UTSUNOMIYA, Daisuke, Ashigarakami-gun, Kanagawa 258-8538 (JP)
(74) Representative: Dehns Germany Partnerschaft mbB
(86) International application number: PCT/JP2020/031845
(87) International publication number: WO 2021/039718

(56) References cited:
- WO-A1-2018/055933
- WO-A1-2018/180249
- JP-A- 2010 175 579
- JP-A- H05 203 882
- US-A1- 2019 204 068

## Description

### BACKGROUND OF THE INVENTION

### 1. Field of the Invention

The present invention relates to an endoscope system that displays a measurement marker to be used to measure the size of a subject and a method of operating the endoscope system.

### 2. Description of the Related Art

A distance to a subject, the size of a subject, and the like are acquired in an endoscope system that includes a light source device, an endoscope, and a processor device. For example, in WO2018/051680A, a subject is irradiated with illumination light and measurement beam light and a beam irradiation region, such as a spot, appears on the subject due to irradiation with beam light. Then, a measurement marker used to measure the size of the subject is displayed in a subject image to correspond to the position of the spot. An endoscope system is described in US 2019/204068, which also discloses the preamble of claim 1.

### SUMMARY OF THE INVENTION

As a method of correctly detecting the position of a spot from a subject image, there is a color separation method using the color/brightness/chroma saturation and the like of an image, such as RGB/HSV. However, in a case where scattered reflection in a living body, residues, or altered (discolored) tissue is included in a subject, the color or the like of an image is changed due to scattered reflection in a living body, or the like. For this reason, it may be difficult to detect the position of the spot. Accordingly, it has been required to stably detect the irradiation region of measurement beam light, such as the position of the spot even in a case where there is scattered reflection in a living body, or the like.

An object of the present invention is to provide an endoscope system that can stably detect an irradiation region of measurement beam light and a method of operating the endoscope system.

An endoscope system according to an aspect of the present invention is provided as defined by claim 1.

It is preferable that the processor recognizes the beam irradiation region by a learning model outputting the beam irradiation region in response to an input of the subject image. It is preferable that the processor recognizes the beam irradiation region by performing pattern matching processing on the subject image using a predetermined template image of the beam irradiation region.

It is preferable that the processor is capable of recognizing the beam irradiation region having a pattern of which the specific shape is deformed.

It is preferable that the specific shape is a circular shape. It is preferable that the specific shape is a linear shape. It is preferable that the processor receives the subject image including a plurality of color images and an amount of the measurement beam light is set to a specific amount of light and a pixel value of each of the color images is set to a maximum pixel value, so that a central region of the beam irradiation region is white.

It is preferable that the processor displays the measurement marker, which corresponds to a position of the beam irradiation region, in the measurement image. It is preferable that the processor displays the measurement marker, which corresponds to a pattern of the beam irradiation region, in the measurement image.

According to another aspect of the present invention, there is provided a method of operating an endoscope system as defined by claim 10.

According to the present invention, it is possible to stably detect the irradiation region of measurement beam light by recognizing a beam irradiation region, which is formed on a subject by the measurement beam light, as a pattern having a specific shape that includes a white central region and a peripheral region.

### BRIEF DESCRIPTION OF THE DRAWINGS

Fig. 1 is a diagram showing the appearance of an endoscope system.
Fig. 2 is a plan view of a distal end part of an endoscope.
Fig. 3 is a block diagram showing the functions of the endoscope system.
Fig. 4 is a graph showing the transmission distribution of a red color filter RF, a green color filter GF, and a blue color filter BF.
Fig. 5 is a block diagram showing the function of a beam light-emitting unit.
Fig. 6 is an image diagram showing a spot SP and a measurement marker.
Fig. 7 is a diagram illustrating a relationship between an optical axis of measurement beam light and an optical axis of an image pickup optical system.
Fig. 8 is a block diagram showing the functions of a signal processing unit.
Fig. 9 is a diagram illustrating a central region CR1 and a peripheral region SR1 of the spot SP.
Fig. 10 is a graph showing the distribution of pixel values of a red image RP, a green image GP, and a blue image BP.
Fig. 11 is a graph showing transmittance in a wavelength range WMB of measurement beam light.
Fig. 12 is a block diagram showing the functions of an irradiation region-recognizing section including a learning model.
Fig. 13 is a diagram illustrating the pattern of the spot SP that is deformed from a circular shape.
Fig. 14 is a block diagram showing the functions of an irradiation region-recognizing section including a pattern matching-processing section.
Fig. 15 is an image diagram showing a spot and a first measurement marker in a case where an observation distance corresponds to a near end Px.
Fig. 16 is an image diagram showing a spot and a first measurement marker in a case where an observation distance corresponds to an intermediate vicinity Py.
Fig. 17 is an image diagram showing a spot and a first measurement marker in a case where an observation distance corresponds to a far end Pz.
Fig. 18 is a diagram illustrating first measurement markers having a cruciform shape, a cruciform shape with gradations, a distorted cruciform shape, a circular-and-cruciform shape, and the shape of a measurement point group.
Fig. 19 is an image diagram showing three concentric circular markers having the same color.
Fig. 20 is an image diagram showing three concentric circular markers having different colors.
Fig. 21 is an image diagram showing distorted concentric circular markers.
Fig. 22 is an image diagram showing an intersection line and gradations.
Fig. 23 is a diagram illustrating a central region CR2 and a peripheral region SR2 of the intersection line.
Fig. 24 is a diagram illustrating a diameter DM of the central region CR1 of the spot SP.
Fig. 25 is a flowchart showing a series of flows of the present invention.

### DESCRIPTION OF THE PREFERRED EMBODIMENTS

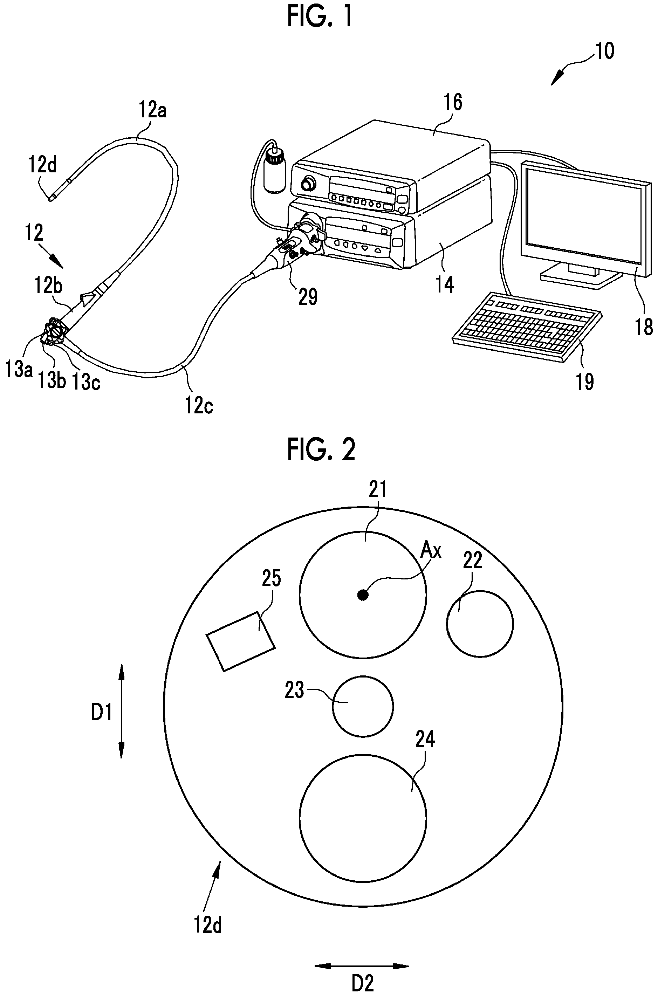

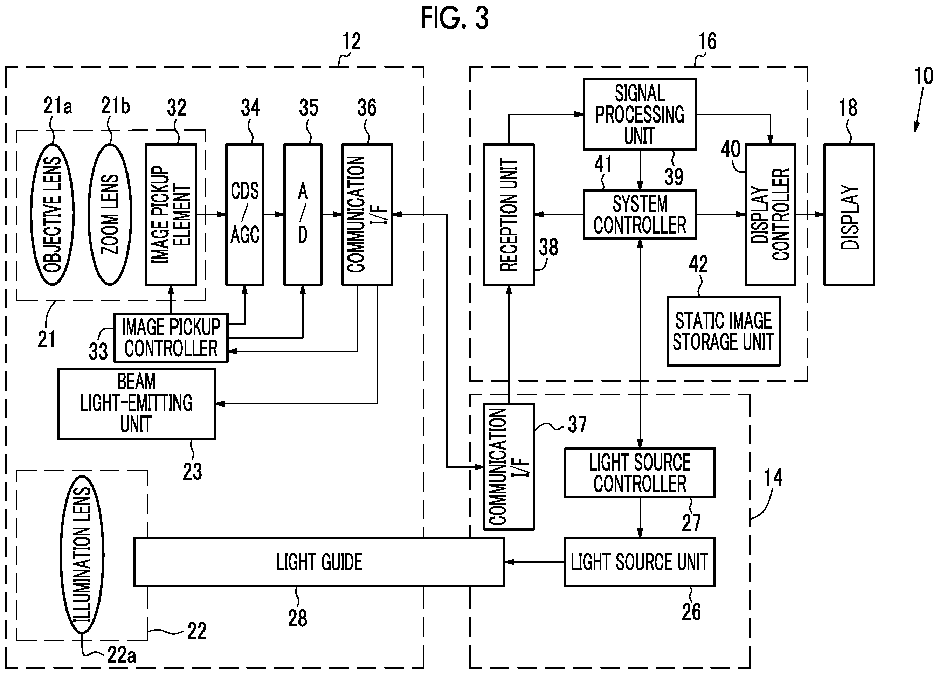

As shown in Fig. 1, an endoscope system 10 includes an endoscope 12, a light source device 14, a processor device 16, a display 18, and a user interface 19. The endoscope 12 includes an insertion part 12a that is to be inserted into an object to be examined, an operation part 12b that is provided at the proximal end portion of the insertion part 12a, and a universal cable 12c. The universal cable 12c is a cable in which a light guide 28 (see Fig. 3) for guiding illumination light emitted from the light source device 14, a control line for transmitting control signals used to control the endoscope 12, a signal line for transmitting image signals obtained from the image pickup of an object to be observed, a power line for supplying power to each part of the endoscope 12, and the like are integrated. The distal end of the universal cable 12c is provided with a connector 29 to be connected to the light source device 14.

The light source device 14 generates illumination light by, for example, a semiconductor light source, such as a light emitting diode (LED) or a laser diode (LD), or a halogen lamp, such as a xenon lamp. In a case where the connector 29 is connected to the light source device 14, illumination light is incident on the light guide 28 (see Fig. 3) of the connector 29 and the object to be observed is irradiated with the illumination light from the distal end of the insertion part 12a.

Further, the light source device 14 is electrically connected to the processor device 16 and the connector 29 of the endoscope 12 is connected to the processor device 16 through the light source device 14. The transmission and reception of control signals, image signals, and the like between the light source device 14 and the connector 29 are performed by wireless communication. For this reason, the light source device 14 transmits the control signals and the like, which are wirelessly transmitted to and received from the connector 29, to the processor device 16. Furthermore, the light source device 14 supplies power, which is used to drive the endoscope 12, to the connector 29, and the supply of this power is also wirelessly performed.

The processor device 16 controls the amount and emission timing of illumination light, which is emitted from the light source device 14, and each part of the endoscope 12 and generates an endoscopic image using image signals that are obtained from the image pickup of an object to be observed irradiated with the illumination light. Further, the processor device 16 is electrically connected to the display 18 and the user interface 19. The display 18 displays the endoscopic image that is generated by the processor device 16, information about the endoscopic image, and the like. The user interface 19 has a function to receive an input operation, such as function settings.

The endoscope 12 has a normal observation mode, a special observation mode, and a length measurement mode, and these three modes are switched by a mode changeover switch 13a that is provided on the operation part 12b of the endoscope 12. The normal observation mode is a mode in which an object to be observed is illuminated with the illumination light. The special observation mode is a mode in which an object to be observed is illuminated with special light different from the illumination light. In the length measurement mode, an object to be observed is illuminated with the illumination light and measurement beam light and a measurement marker to be used for the measurement of the size and the like of an object to be observed is displayed in a subject image obtained from the image pickup of the object to be observed. The illumination light is light that is used to observe the entire object to be observed by applying brightness to the entire object to be observed. The special light is light that is used to emphasize a specific region of the object to be observed. The measurement beam light is light that is used for the display of the measurement marker.

Further, the operation part 12b of the endoscope 12 is provided with a freeze switch 13b that is used to give a static image-acquisition instruction to acquire the static image of a subject image. In a case where a user operates the freeze switch 13b, the screen of the display 18 is frozen and displayed and an alert sound (for example, "beep") informing the acquisition of a static image is generated together. Then, the static images of the subject image, which are obtained before and after the operation timing of the freeze switch 13b, are stored in a static image storage unit 42 (see Fig. 3) provided in the processor device 16. The static image storage unit 42 is a storage unit, such as a hard disk or a universal serial bus (USB) memory. In a case where the processor device 16 can be connected to a network, the static image of the subject image may be stored in a static image storage server (not shown), which is connected to the network, instead of or in addition to the static image storage unit 42.

A static image-acquisition instruction may be given using an operation device other than the freeze switch 13b. For example, a foot pedal may be connected to the processor device 16, and a static image-acquisition instruction may be given in a case where a user operates the foot pedal (not shown) with a foot. A static image-acquisition instruction may be given by a foot pedal that is used to switch a mode. Further, a gesture recognition unit (not shown), which recognizes the gestures of a user, may be connected to the processor device 16, and a static image-acquisition instruction may be given in a case where the gesture recognition unit recognizes a specific gesture of a user. The gesture recognition unit may also be used to switch a mode.

Further, a sight line input unit (not shown), which is provided close to the display 18, may be connected to the processor device 16, and a static image-acquisition instruction may be given in a case where the sight line input unit recognizes that a user's sight line is in a predetermined region of the display 18 for a predetermined time or longer. Furthermore, a voice recognition unit (not shown) may be connected to the processor device 16, and a static image-acquisition instruction may be given in a case where the voice recognition unit recognizes a specific voice generated by a user. The voice recognition unit may also be used to switch a mode. Moreover, an operation panel (not shown), such as a touch panel, may be connected to the processor device 16, and a static image-acquisition instruction may be given in a case where a user performs a specific operation on the operation panel. The operation panel may also be used to switch a mode.

As shown in Fig. 2, a distal end part 12d of the endoscope 12 has a substantially circular shape, and is provided with an image pickup optical system 21 that receives light from a subject, an illumination optical system 22 that is used to irradiate the subject with the illumination light, a beam light-emitting unit 23 that radiates the measurement beam light to the subject, an opening 24 that allows a treatment tool to protrude toward the subject, and an air/water supply nozzle 25 that is used to supply air and water.

An optical axis Ax of the image pickup optical system 21 extends in a direction perpendicular to the plane of paper. A vertical first direction D1 is orthogonal to the optical axis Ax, and a horizontal second direction D2 is orthogonal to the optical axis Ax and the first direction D1. The image pickup optical system 21 and the beam light-emitting unit 23 are provided at different positions in the distal end part 12d, and are arranged in the first direction D1.

As shown in Fig. 3, the light source device 14 comprises a light source unit 26 and a light source controller 27. The light source unit 26 generates the illumination light or the special light that is used to illuminate the subject. The illumination light or the special light, which is emitted from the light source unit 26, is incident on the light guide 28, and the subject is irradiated with the illumination light or the special light through an illumination lens 22a. A white light source emitting white light, a plurality of light sources, which include a white light source and a light source emitting another color light (for example, a blue light source emitting blue light), or the like is used as a light source of the illumination light in the light source unit 26. Further, a light source, which emits broadband light including blue narrow-band light used to emphasize superficial information about superficial blood vessels and the like, is used as a light source of the special light in the light source unit 26. The light source controller 27 is connected to a system controller 41 of the processor device 16. Mixed white light, which is a combination of blue light, green light, and red light, may be used as the illumination light. In this case, it is preferable that the illumination optical system 22 is optically designed to allow the irradiation range of green light to be wider than the irradiation range of red light.

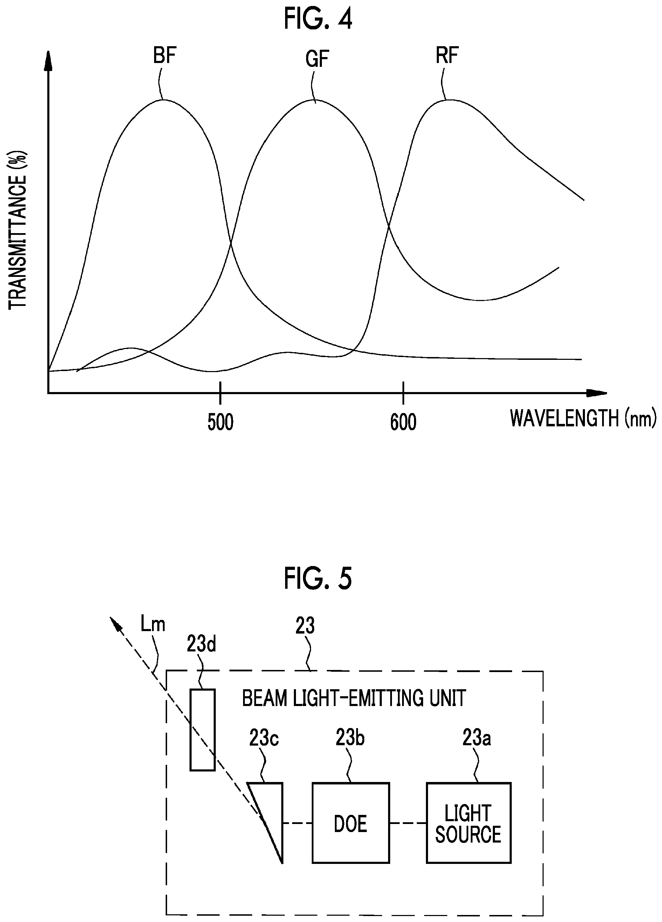

The light source controller 27 controls the light source unit 26 on the basis of an instruction given from the system controller 41. The system controller 41 not only gives an instruction related to light source control to the light source controller 27 but also controls a light source 23a (see Fig. 5) of the beam light-emitting unit 23. In the case of the normal observation mode, the system controller 41 performs a control to turn on the illumination light and to turn off the measurement beam light. In the case of the special observation mode, the system controller 41 performs a control to turn on the special light and to turn off the measurement beam light. In the case of the length measurement mode, the system controller 41 performs a control to turn on the illumination light and to turn on the measurement beam light.

The illumination optical system 22 includes the illumination lens 22a, and the object to be observed is irradiated with light, which is emitted from the light guide 28, through the illumination lens 22a. The image pickup optical system 21 includes an objective lens 21a, a zoom lens 21b, and an image pickup element 32. Light reflected from the object to be observed is incident on the image pickup element 32 through the objective lens 21a and the zoom lens 21b. Accordingly, the reflected image of the object to be observed is formed on the image pickup element 32.

The zoom lens 21b has an optical zoom function to enlarge or reduce the subject as a zoom function by moving between a telephoto end and a wide end. ON/OFF of the optical zoom function can be switched by a zoom operation part 13c (see Fig. 1) provided on the operation part 12b of the endoscope, and the subject can be enlarged or reduced at a specific magnification ratio in a case where the zoom operation part 13c is further operated in a state where the optical zoom function is ON.

The image pickup element 32 is a color image pickup sensor, and picks up the reflected image of an object to be examined and outputs image signals. It is preferable that the image pickup element 32 is a charge coupled device (CCD) image pickup sensor, a complementary metal-oxide semiconductor (CMOS) image pickup sensor, or the like. The image pickup element 32 used in an embodiment of the present invention is a color image pickup sensor that is used to obtain red images, green images, and blue images corresponding to three colors of R (red), G (green), and B (blue). The red image is an image that is output from red pixels provided with red color filters in the image pickup element 32. The green image is an image that is output from green pixels provided with green color filters in the image pickup element 32. The blue image is an image that is output from blue pixels provided with blue color filters in the image pickup element 32. The image pickup element 32 is controlled by an image pickup controller 33.

Since a red color filter RF has high transmittance in a red-light wavelength range of 600 to 700 nm as shown in Fig. 4, a red pixel has high sensitivity in the red-light wavelength range. Since a green color filter GF has high transmittance in a green-light wavelength range of 500 to 600 nm, a green pixel has high sensitivity in the green-light wavelength range. Since a blue color filter BF has high transmittance in a blue-light wavelength range of 400 to 500 nm, a blue pixel has high sensitivity in the blue-light wavelength range. A red pixel has sensitivity even in the green-light wavelength range or the blue-light wavelength range. Further, a green pixel has sensitivity even in the red-light wavelength range or the blue-light wavelength range. Furthermore, a blue pixel has sensitivity even in the red-light wavelength range or the green-light wavelength range.

Image signals output from the image pickup element 32 are transmitted to a CDS/AGC circuit 34. The CDS/AGC circuit 34 performs correlated double sampling (CDS) or auto gain control (AGC) on the image signals that are analog signals. The image signals, which have been transmitted through the CDS/AGC circuit 34, are converted into digital image signals by an analog/digital converter (A/D converter) 35. The digital image signals, which have been subjected to A/D conversion, are input to a communication interface (I/F) 37 of the light source device 14 through a communication interface (I/F) 36.

In the processor device 16, programs related to various types of processing are incorporated into a program memory (not shown). The programs incorporated into the program memory are operated by the system controller 41 formed of a processor, so that the processor device 16 realizes the functions of a reception unit 38, a signal processing unit 39, and a display controller 40.

The reception unit 38 is connected to the communication interface (I/F) 37 of the light source device 14. The reception unit 38 receives the image signals, which are transmitted from the communication I/F 37, and transmits the image signals to the signal processing unit 39. A memory, which temporarily stores the image signals received from the reception unit 38, is built in the signal processing unit 39, and the signal processing unit 39 processes an image signal group, which is a set of the image signals stored in the memory, to generate the subject image. The reception unit 38 may directly transmit control signals, which are related to the light source controller 27, to the system controller 41. Further, a processing unit or a storage unit (for example, an irradiation region-recognizing section 58 or a marker table 62), which are related to the length measurement mode, of the processor device 16 may be provided in a length measurement processor (not shown) separate from the processor device 16. In this case, the length measurement processor and the processor device 16 are in a state where the length measurement processor and the processor device 16 can communicate with each other so that images or various types of information can be transmitted and received.

In a case where the endoscope 12 is set to the normal observation mode, signal assignment processing for assigning the blue image of the subject image to B channels of the display 18, assigning the green image of the subject image to G channels of the display 18, and assigning the red image of the subject image to R channels of the display 18 is performed in the signal processing unit 39. As a result, a color subject image is displayed on the display 18. The same signal assignment processing as that in the normal observation mode is performed even in the length measurement mode. On the other hand, in a case where the endoscope 12 is set to the special observation mode, the red image of the subject image is not used for the display of the display 18, the blue image of the subject image is assigned to the B channels and the G channels of the display 18, and the green image of the subject image is assigned to the R channels of the display 18 in the signal processing unit 39. As a result, a pseudo color subject image is displayed on the display 18. In a case where the endoscope 12 is set to the length measurement mode, the signal processing unit 39 may be adapted to perform structure-emphasis processing for emphasizing structures, such as blood vessels, or color difference-emphasis processing for increasing a color difference between a normal area and a lesion area of the object to be observed on the subject image.

The display controller 40 causes the display 18 to display the subject image that is generated by the signal processing unit 39. The system controller 41 performs the control of the image pickup element 32 through the image pickup controller 33 provided in the endoscope 12. The image pickup controller 33 also performs the control of the CDS/AGC circuit 34 and the A/D converter 35 together with the control of the image pickup element 32.

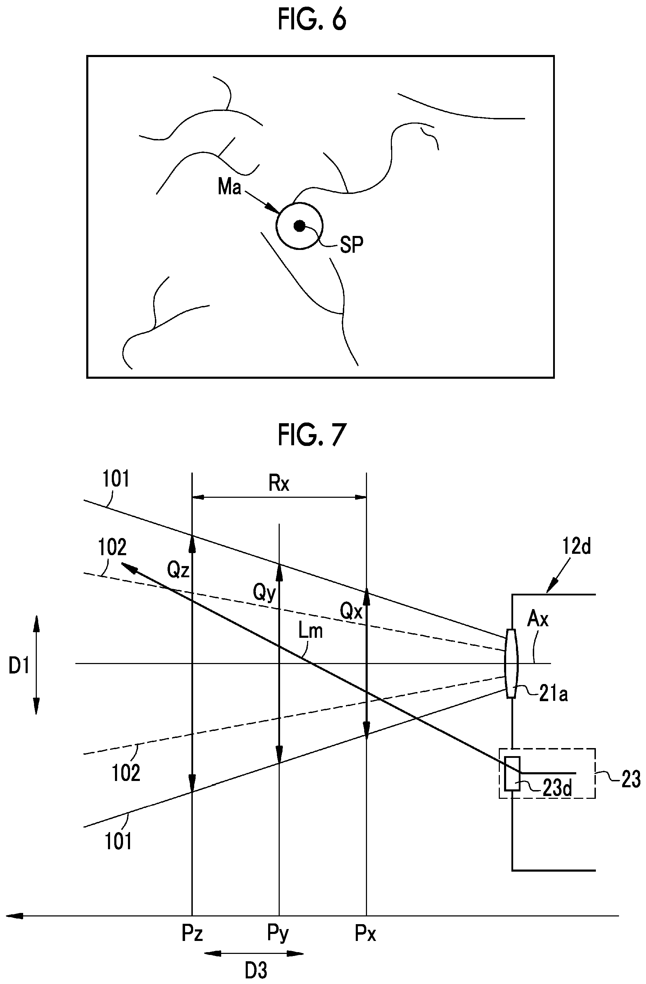

As shown in Fig. 5, the beam light-emitting unit 23 emits measurement beam light Lm obliquely with respect to the optical axis Ax (see Fig. 7) of the image pickup optical system 21. The beam light-emitting unit 23 comprises a light source 23a, a diffractive optical element (DOE) 23b, a prism 23c, and an emitting section 23d. The light source 23a is to emit light having a color that can be detected by pixels of the image pickup element 32 (specifically visible light), and includes a light emitting element, such as a laser light source (laser diode (LD)) or a light emitting diode (LED), and a condenser lens that condenses light emitted from the light emitting element. The light source 23a is provided on a scope electric board (not shown). The scope electric board is provided in the distal end part 12d of the endoscope, and receives power supplied from the light source device 14 or the processor device 16 and supplies power to the light source 23a.

For example, red (the color of beam light) laser light having a wavelength of 600 nm or more and 650 nm or less is used as the light emitted from the light source 23a in this embodiment, but light having a wavelength in other ranges, for example, green light having a wavelength of 495 nm or more and 570 nm or less or blue light may be used. The light source 23a is controlled by the system controller 41, and emits light on the basis of an instruction given from the system controller 41. The DOE 23b converts the light, which is emitted from the light source, into the measurement beam light that is used to obtain measurement information. It is preferable that the amount of measurement beam light is adjusted from a standpoint of protecting a human body, eyes, and internal organs and is adjusted to the amount of light enough to cause halation (pixel saturation) in the observation range of the endoscope 12.

The prism 23c is an optical member that is used to change the travel direction of the measurement beam light converted by the DOE 23b. The prism 23c changes the travel direction of the measurement beam light so that the measurement beam light intersects with the visual field of the image pickup optical system 21 including the objective lens 21a. The details of the travel direction of the measurement beam light will also be described later. A subject is irradiated with the measurement beam light Lm, which is emitted from the prism 23c, through the emitting section 23d that is formed of an optical member.

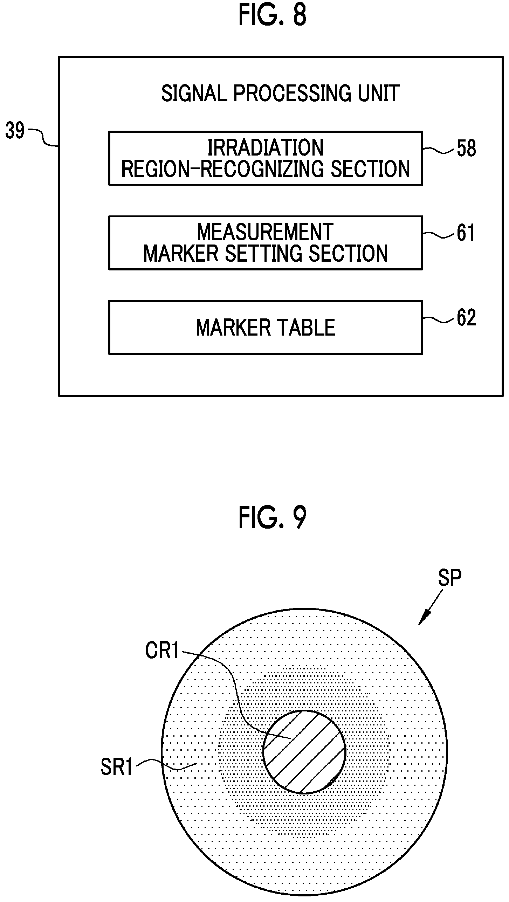

In a case where the subject is irradiated with the measurement beam light, a spot SP as a beam irradiation region is formed on the subject as shown in Fig. 6. The position of the spot SP is recognized by the irradiation region-recognizing section 58 (see Fig. 8), and a measurement marker showing the size of the subject is set according to the position of the spot SP. The display controller 40 causes the display 18 to display a measurement image in which the set measurement marker is displayed in the subject image.

The position of the spot SP and an observation distance (a distance between the distal end part 12d of the endoscope and the object to be observed) are related to each other. The observation distance is shorter as the position of the spot SP is closer to the lower side, and the observation distance is longer as the position of the spot SP is closer to the upper side. The set measurement marker Ma is displayed in the subject image. The radius of the measurement marker Ma from the spot SP shows the actual size (for example, 5 mm) of the subject. Further, the size (radius) of the measurement marker Ma is changed depending on the position of the spot SP, that is, the observation distance. For example, the size of the measurement marker Ma is larger as the position of the spot SP is closer to the lower side, and the size of the measurement marker Ma is smaller as the position of the spot SP is closer to the upper side.

A plurality of types of measurement markers, such as a first measurement marker and a second measurement marker, are included in the measurement marker as described later, and a measurement marker to be displayed in the subject image among the plurality of types of measurement markers can be selected according to a user's instruction. For example, the user interface 19 is used for the user's instruction.

The emitting section 23d may be formed of an auxiliary measurement slit, which is formed at the distal end part 12d of the endoscope, instead of being formed of an optical member. Further, in a case where the emitting section 23d is formed of an optical member, it is preferable that an anti-reflection coating (AR coating) (anti-reflection portion) is provided on the emitting section 23d. The reason why the anti-reflection coating is provided as described above is that it is difficult for the irradiation region-recognizing section 58 to be described later to recognize the position of the spot SP formed on the subject by the measurement beam light in a case where the measurement beam light is reflected without being transmitted through the emitting section 23d and a ratio of the measurement beam light with which a subject is irradiated is reduced.

The beam light-emitting unit 23 has only to be capable of emitting the measurement beam light to the visual field of the image pickup optical system 21. For example, the light source 23a may be provided in the light source device and light emitted from the light source 23a may be guided to the DOE 23b by optical fibers. Further, the prism 23c may not be used and the orientations of the light source 23a and the DOE 23b may be inclined with respect to the optical axis Ax of the image pickup optical system 21 so that the measurement beam light Lm is emitted in a direction crossing the visual field of the image pickup optical system 21.

With regard to the travel direction of the measurement beam light, the measurement beam light is emitted in a state where the optical axis of the measurement beam light Lm intersects with the optical axis Ax of the image pickup optical system 21 as shown in Fig. 7. In a case where the subject can be observed in a range Rx of an observation distance, it is understood that the positions (points where the respective arrows Qx, Qy, and Qz intersect with the optical axis of the measurement beam light Lm) of the spot SP, which is formed on the subject by the measurement beam light Lm, in image pickup ranges (shown by arrows Qx, Qy, and Qz) at a near end Px, an intermediate vicinity Py, and a far end Pz of the range Rx are different from each other. The image pickup angle of view of the image pickup optical system 21 is represented by a region between two solid lines 101, and measurement is performed in a central region (a region between two dotted lines 102), in which an aberration is small, of this image pickup angle of view.

Since the measurement beam light Lm is emitted in a state where the optical axis of the measurement beam light Lm intersects with the optical axis Ax as described above, the size of the subject can be measured from the movement of the position of the spot with respect to a change in observation distance. Then, the image of the subject illuminated with the measurement beam light is picked up by the image pickup element 32, so that a subject image including the spot SP is obtained. In the subject image, the position of the spot SP varies depending on a relationship between the optical axis Ax of the image pickup optical system 21 and the optical axis of the measurement beam light Lm and an observation distance. However, the number of pixels showing the same actual size (for example, 5 mm) is increased in the case of a short observation distance, and the number of pixels showing the same actual size is reduced in the case of a long observation distance.

As shown in Fig. 8, the signal processing unit 39 of the processor device 16 comprises an irradiation region-recognizing section 58, a measurement marker setting section 61, and a marker table 62. In a case where the endoscope 12 is set to the normal observation mode, the subject image of the subject illuminated with the illumination light is input to the signal processing unit 39. In a case where the endoscope 12 is set to the special observation mode, the subject image of the subject illuminated with the special light is input to the signal processing unit 39. In a case where the endoscope 12 is set to the length measurement mode, the subject image of the subject illuminated with the illumination light and the measurement beam light is input to the signal processing unit 39.

The irradiation region-recognizing section 58 recognizes a beam irradiation region, which has a pattern having a specific shape, from the subject image. Specifically, the pattern having a specific shape includes a white central region CR1 and a peripheral region SR1 that covers the periphery of the central region and has a feature quantity based on the measurement beam light. In a case where the beam irradiation region is the above-mentioned spot SP, the pattern having a specific shape has a circular shape as shown in Fig. 9. In this case, the white central region CR1 has a circular shape, and the peripheral region SR1 has the shape of a ring.

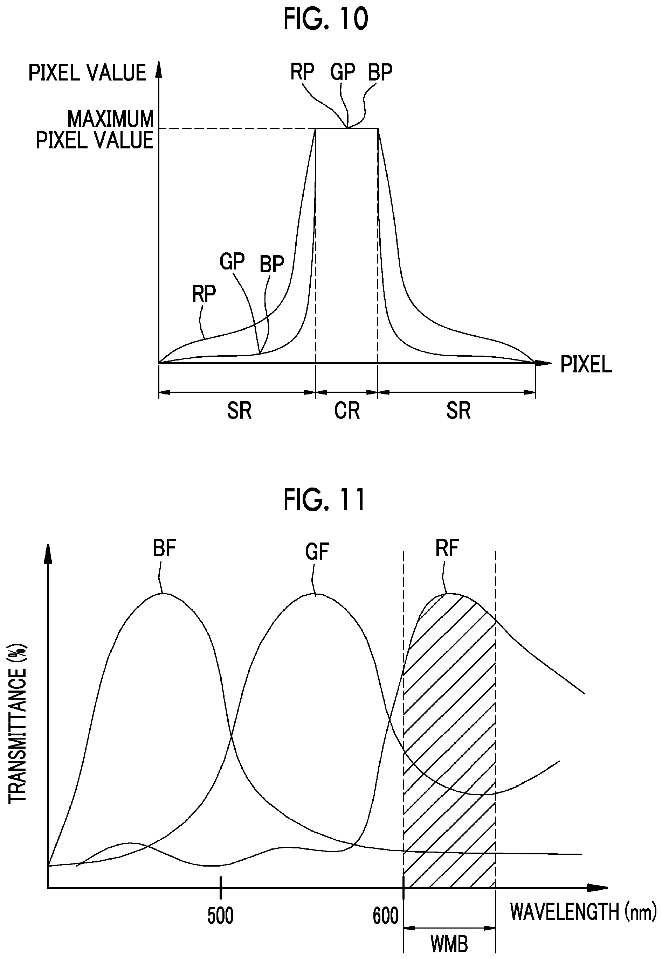

Fig. 10 shows the distribution of pixel values of the respective color images of the subject image that includes a red image RP, a green image GP, and a blue image BP as a plurality of color images. Since the pixel values of the red image RP, the green image GP, and the blue image in the central region CR1 reach the maximum pixel value (for example, 255), the central region CR1 is white. In a case where the measurement beam light is incident on the image pickup element 32 in this case, not only the red color filter RF of the image pickup element 32 but also all the green color filter GF and the blue color filter BF transmit the measurement beam light with a transmittance of 100% in the wavelength range WMB of the measurement beam light as shown in Fig. 11. On the other hand, the pixel value of the red image RP is larger than the pixel value of the green image GP or the blue image BP in the peripheral region SR1. For this reason, the peripheral region SR1 has redness. The measurement beam light is emitted with a specific amount of light in the light source unit 26, so that the pixel values of the red image RP, the green image GP, and the blue image BP in the central region CR1 are set to the maximum pixel value.

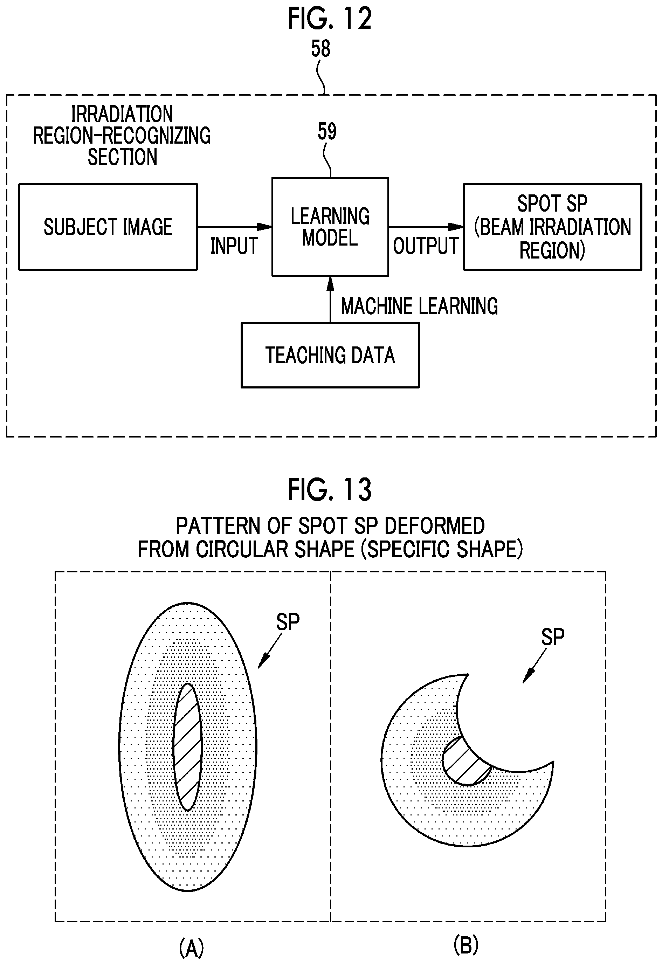

The irradiation region-recognizing section 58 can recognize the spot SP that has the specific shape and the feature quantity described above. Specifically, it is preferable that the irradiation region-recognizing section 58 includes a learning model 59 for recognizing the spot SP by outputting the spot SP, which is a beam irradiation region, in response to the input of the subject image as shown in Fig. 12. The learning model 59 is subjected to machine learning using many teaching data in which a subject image and beam irradiation regions having been already recognized are associated with each other. It is preferable that a convolutional neural network (CNN) is used as the machine learning.

Since the spot SP is recognized using the learning model 59, not only the circular spot SP (see Fig. 9) that is formed of the circular central region CR1 and the ring-shaped peripheral region SR1 but also spots SP that have patterns deformed from a circular shape, which is a specific shape, can be recognized. For example, a spot SP, which is deformed in a vertical direction as shown in (A) of Fig. 13, can also be recognized. Further, a spot SP, which is deformed such that a part of a circular shape is cut out as shown in (B) of Fig. 13, can also be recognized. Furthermore, the feature quantity of the peripheral region SR1, which can be recognized by the learning model 59, includes a blue color or a green color other than a red color that is the color of the measurement beam light. Moreover, the feature quantity of the peripheral region SR1, which can be recognized by the learning model 59, includes the luminance, brightness, chroma saturation, or hue of the measurement beam light. It is preferable that luminance conversion processing or processing for converting brightness, chroma saturation, or a hue is performed on the peripheral region of the spot SP included in the subject image to acquire the luminance, brightness, chroma saturation, or hue of the measurement beam light.

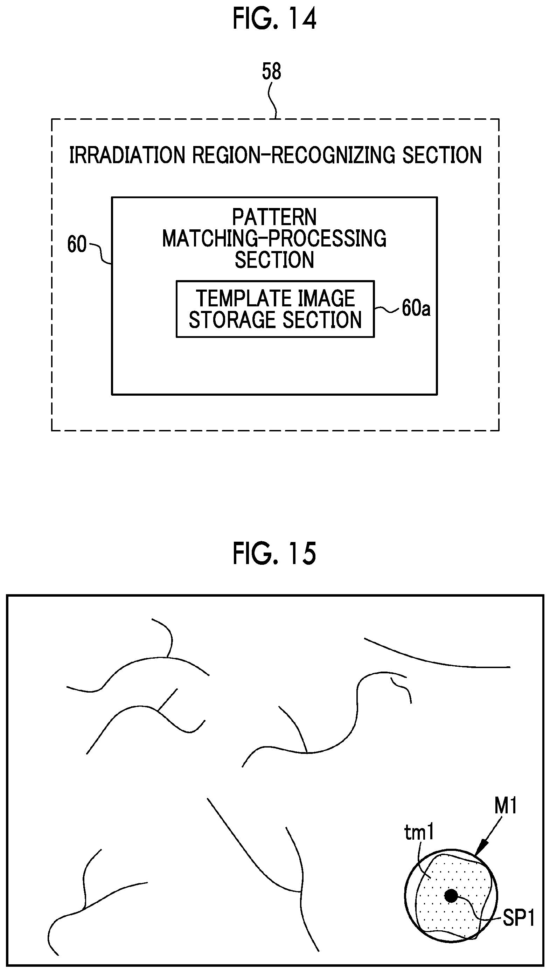

Further, as shown in Fig. 14, the irradiation region-recognizing section 58 may include a pattern matching-processing section 60 that recognizes the spot SP by performing pattern matching processing on the subject image using the template image of the spot SP as a predetermined template image of a beam irradiation region. The template image of the spot SP is stored in a template image storage section 60a. Not only the template image of the circular spot SP that is formed of the circular central region CR1 and the ring-shaped peripheral region SR1 but also the template images of spots SP that have patterns deformed from a circular shape, which is a specific shape, are stored in the template image storage section 60a. Further, the pattern matching-processing section 60 can also perform pattern matching processing using the pattern of the feature quantity of the peripheral region SR1. A feature quantity, which can be subjected to pattern matching processing, includes the distribution of a blue color or a green color other than the distribution of a red color that is the color of the measurement beam light. Further, a feature quantity, which can be subjected to pattern matching processing, includes the distribution of the luminance, brightness, chroma saturation, or hue of the measurement beam light.

The measurement marker setting section 61 sets a first measurement marker, which shows the actual size (the same size as the actual size) of the subject, as a measurement marker, which is used to measure the size of the subject, on the basis of the position of the spot SP. The measurement marker setting section 61 sets a measurement marker image, which corresponds to the position of the spot SP, with reference to the marker table 62 in which a measurement marker image of which the display aspect varies depending on the irradiation position of the spot SP is stored in association with the position of the spot SP. In addition to the position of the spot SP, the measurement marker image of which the display aspect varies depending on a marker display position where the first measurement marker is displayed on the display 18 may be stored in the marker table 62 in association with the position of the spot SP and the marker display position.

For example, the size or shape of the measurement marker image varies depending on the irradiation position of the spot SP. The display of the measurement marker image will be described later. Further, the contents stored in the marker table 62 are maintained even in a case where the power of the processor device 16 is turned off. The measurement marker image and the irradiation position are stored in the marker table 62 in association with each other, but a distance to the subject (a distance between the distal end part 12d of the endoscope 12 and the subject) corresponding to the irradiation position and the measurement marker image may be stored in the marker table 62 in association with each other.

The display controller 40 causes the display 18 to display the measurement image in which a measurement marker is displayed in the subject image on the basis of the position of the spot SP that is a beam irradiation region. Specifically, the display controller 40 causes the display 18 to display the measurement image in which the first measurement marker is superimposed to center on the spot SP. For example, a circular measurement marker is used as the first measurement marker. In this case, as shown in Fig. 15, a marker M1, which shows an actual size of 5 mm (a horizontal direction and a vertical direction of the subject image), is displayed at the center of a spot SP1 formed on a tumor tm1 of a subject in a case where an observation distance is close to the near end Px. In a case where the measurement marker is displayed on the display 18, an observation distance may also be displayed on the display 18 together.

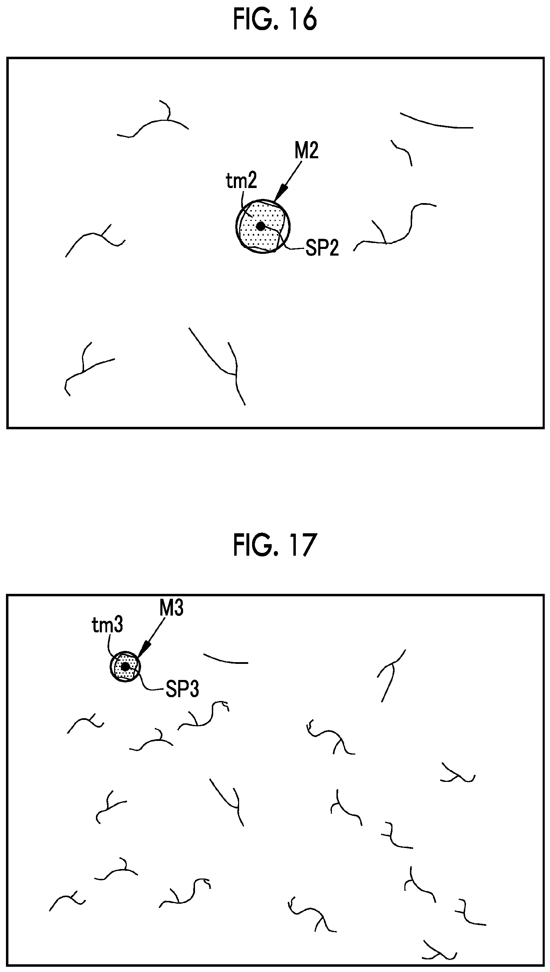

Further, as shown in Fig. 16, a marker M2, which shows an actual size of 5 mm (the horizontal direction and the vertical direction of the subject image), is displayed at the center of a spot SP2 formed on a tumor tm2 of a subject in a case where an observation distance is close to the intermediate vicinity Py. Since the marker display position of the marker M2 is positioned at the central portion of the subject image that is less likely to be affected by distortion caused by the image pickup optical system 21, the marker M2 has a circular shape without being affected by the distortion or the like.

Furthermore, as shown in Fig. 17, a marker M3, which shows an actual size of 5 mm (the horizontal direction and the vertical direction of the subject image), is displayed at the center of a spot SP3 formed on a tumor tm3 of a subject. As shown in Figs. 15 to 17 having been described above, the size of the first measurement marker corresponding to the same actual size of 5 mm is reduced with an increase in an observation distance. Moreover, the shape of the first measurement marker also varies depending on the marker display position due to an influence of the distortion caused by the image pickup optical system 21.

In Figs. 15 to 17, the center of the spot SP and the center of the marker are displayed to coincide with each other. However, the first measurement marker may be displayed at a position away from the spot SP in a case where there is no problem in measurement accuracy. Even in this case, it is preferable that the first measurement marker is displayed near the spot.

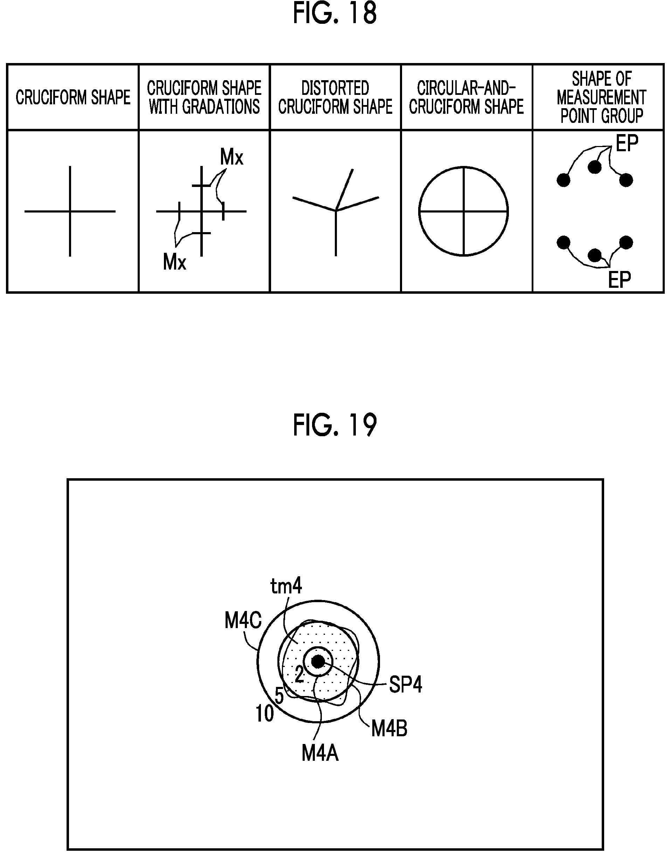

The first measurement marker corresponding to the actual size of the subject, which is 5 mm, is displayed in Figs. 15 to 17, but the actual size of the subject may be set to any value (for example, 2 mm, 3 mm, 10 mm, or the like) according to an object to be observed or the purpose of observation. Further, the first measurement marker has a substantially circular shape in Figs. 15 to 17, but may have a cruciform shape where a vertical line and a horizontal line intersect with each other as shown in Fig. 18. Furthermore, the first measurement marker may have a cruciform shape with gradations where gradations Mx are given to at least one of a vertical line or a horizontal line of a cruciform shape. Further, the first measurement marker may have a distorted cruciform shape of which at least one of a vertical line or a horizontal line is inclined. Furthermore, the first measurement marker may have a circular-and-cruciform shape where a cruciform shape and a circle are combined with each other. In addition, the first measurement marker may have the shape of a measurement point group where a plurality of measurement points EP corresponding to an actual size from a spot are combined with each other. Further, one first measurement marker may be displayed or a plurality of first measurement markers may be displayed, and the color of the first measurement marker may be changed according to an actual size.

As shown in Fig. 19, three concentric circular markers M4A, M4B, and M4C having different sizes (diameters as the sizes are 2 mm, 5 mm, and 10 mm, respectively) may be displayed in the subject image as the first measurement marker to center on a spot SP4 formed on a tumor tm4. Since the three concentric circular markers are displayed as a plurality of markers, time and effort required to switch a marker can be saved and measurement can be performed even in a case where a subject has a non-linear shape. In a case where a plurality of concentric circular markers are to be displayed to center on a spot, a size and a color are not designated for each marker and combinations of a plurality of conditions may be prepared in advance and one can be selected from these combinations.

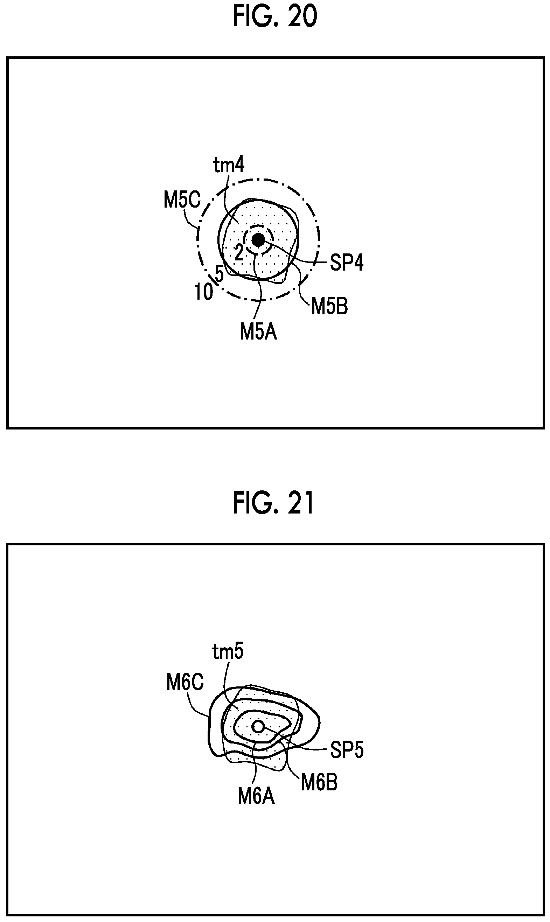

In Fig. 19, all the three concentric circular markers are displayed with the same color (black). However, in a case where a plurality of concentric circular markers are to be displayed, a plurality of color concentric circular markers of which colors are different from each other may be used. As shown in Fig. 20, a marker M5A is displayed by a dotted line representing a red color, a marker M5B is displayed by a solid line representing a blue color, and a marker M5C is displayed by a one-dot chain line representing a white color. Since identifiability can be improved in a case where the colors of the markers are different from each other in this way, measurement can be easily performed.

Further, as shown in Fig. 21, a plurality of distorted concentric circular markers, which are distorted from the respective concentric circles, may be used as the first measurement marker other than the plurality of concentric circular markers. In this case, distorted concentric circular markers M6A, M6B, and M6C are displayed in the subject image to center on a spot SP5 formed on a tumor tm5.

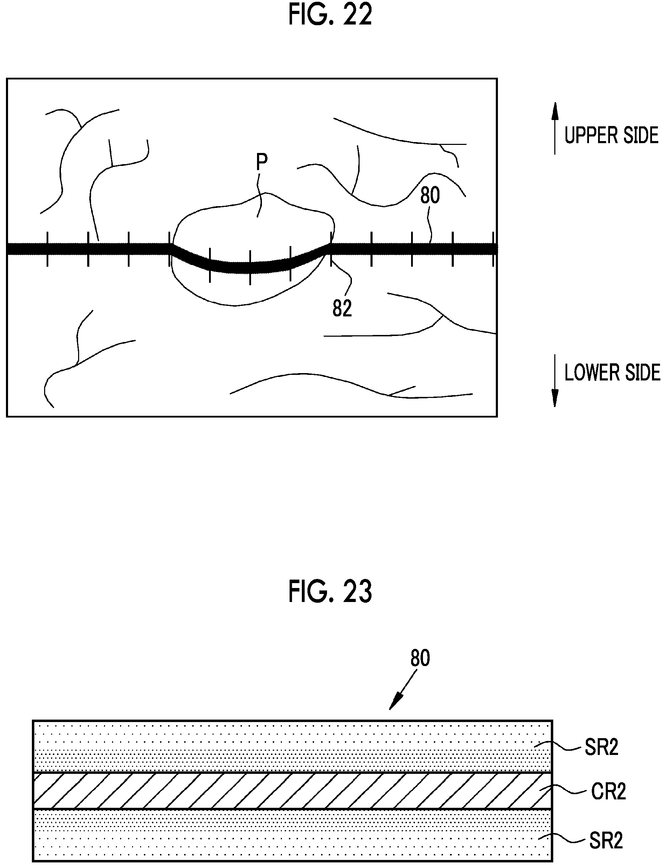

Light, which forms a spot in a case where a subject is irradiated with the light, is used as the measurement beam light, but other light may be used. For example, plane measurement beam light, which forms an intersection line 80 on the subject as shown in Fig. 22 in a case where the subject is irradiated with the light, may be used. In a case where the subject is irradiated with the plane measurement beam light, the intersection line 80 as a line-like beam irradiation region is formed on the subject. In this case, a second measurement marker that consists of the intersection line 80 and gradations 82 formed on the intersection line 80 and serving as an index of the size of the subject (for example, a polyp P) is generated as a measurement marker.

In a case where the plane measurement beam light is used, the irradiation region-recognizing section 58 recognizes the position of the intersection line 80 (the irradiation position of the measurement beam light) as the beam irradiation region that has the pattern having a specific shape. As shown in Fig. 23, the intersection line 80 includes a white central region CR2 and a peripheral region SR2 that covers the white central region R2 and has a feature quantity based on the measurement beam light. The central region CR2 has a linear shape, and the peripheral region SR2 is formed in the shape of two lines that are spaced from each other with a specific interval therebetween. The irradiation region-recognizing section 58 can recognize the intersection line 80 that has the pattern and the feature quantity described above. An intersection line 80 that has a shape deformed from a linear shape, such as a shape in which a part or the like of a line is cut out can also be recognized by the irradiation region-recognizing section 58.

Like the spot SP, the observation distance is shorter as the intersection line 80 recognized by the irradiation region-recognizing section 58 is positioned closer to the lower side, and the observation distance is longer as the intersection line 80 is positioned closer to the upper side. For this reason, in a case where the second measurement marker is displayed in the measurement image, an interval between the gradations 82 is larger as the intersection line 80 is positioned closer to the lower side and an interval between the gradations 82 is smaller as the intersection line 80 is positioned closer to the upper side.

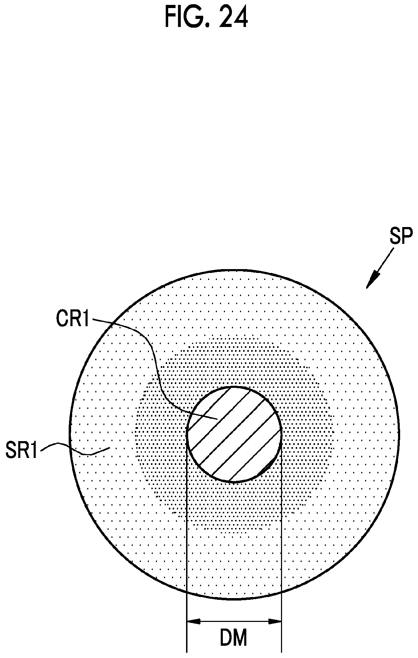

In the above description, a measurement marker corresponding to the position of a beam irradiation region, such as the spot SP or the intersection line 80, has been displayed in the measurement image. However, a measurement marker corresponding to the pattern of a beam irradiation region may be displayed in the measurement image instead of this. For example, in a case where a beam irradiation region is the spot SP, a diameter DM of the white central region CR1 of the spot SP is obtained as shown in Fig. 24 and a measurement marker corresponding to the diameter DM is displayed in the measurement image. It is understood that the diameter DM is large in a case where an observation distance is short, and the diameter is reduced as an observation distance is increased. A measurement marker image of which the size, shape, or the like varies depending on the diameter DM is determined using a relationship between the diameter DM and an observation distance.

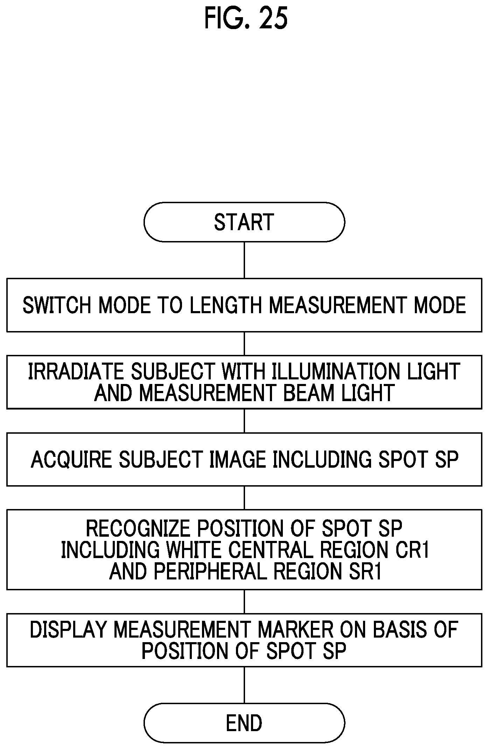

Next, a series of flows of the present invention will be described with reference to a flowchart shown in Fig. 25. In a case where a user operates the mode changeover switch 13a to switch a mode to the length measurement mode, a subject is irradiated with the illumination light and the measurement beam light. A spot SP is formed on the subject by the measurement beam light. A subject image including the spot SP is obtained from the image pickup of the subject including the spot SP.

The reception unit 38 receives the subject image output from the endoscope 12. The irradiation region-recognizing section 58 recognizes the position of the spot SP from the subject image. In this case, the irradiation region-recognizing section 58 recognizes the position of the spot SP that includes the white central region CR1 and the peripheral region SR1 covering the periphery of the central region CR1 and having a feature quantity based on the measurement beam light. The display controller 40 causes the display 18 to display a measurement image in which a measurement marker to be used to measure the size of the subject is displayed in the subject image on the basis of the position of the spot SP recognized by the irradiation region-recognizing section 58. The measurement marker corresponds to the position of the spot SP, and the size, shape, or the like is changed depending on the position of the spot SP.

In the embodiment, the hardware structures of processing units, which perform various types of processing, such as the reception unit 38, the signal processing unit 39, the display controller 40, the system controller 41, the static image storage unit 42, the irradiation region-recognizing section 58, the learning model 59, the pattern matching-processing section 60, the template image storage section 60a, the measurement marker setting section 61, and the marker table 62, are various processors to be described below. Various processors include: a central processing unit (CPU) that is a general-purpose processor functioning as various processing units by executing software (program); a programmable logic device (PLD) that is a processor of which the circuit configuration can be changed after manufacture, such as a field programmable gate array (FPGA); a dedicated electrical circuit that is a processor having circuit configuration designed exclusively to perform various types of processing; and the like.

One processing unit may be formed of one of these various processors, or may be formed of a combination of two or more same kind or different kinds of processors (for example, a plurality of FPGAs, or a combination of a CPU and an FPGA). Further, a plurality of processing units may be formed of one processor. As an example where a plurality of processing units are formed of one processor, first, there is an aspect where one processor is formed of a combination of one or more CPUs and software as typified by a computer, such as a client or a server, and functions as a plurality of processing units. Second, there is an aspect where a processor fulfilling the functions of the entire system, which includes a plurality of processing units, by one integrated circuit (IC) chip as typified by System On Chip (SoC) or the like is used. In this way, various processing units are formed using one or more of the above-mentioned various processors as hardware structures.

In addition, the hardware structures of these various processors are more specifically electrical circuitry where circuit elements, such as semiconductor elements, are combined. Further, the hardware structure of the storage unit is a storage device, such as a hard disc drive (HDD) or a solid state drive (SSD).

### Explanation of References

10: endoscope system
12: endoscope
12a: insertion part
12b: operation part
12c: bendable part
12d: distal end part
13a: mode changeover switch
13b: freeze switch
13c: zoom operation part
14: light source device
16: processor device
18: display
19: user interface
21: image pickup optical system
21a: objective lens
21b: zoom lens
22: illumination optical system
22a: illumination lens
23: beam light-emitting unit
23a: light source
23b: DOE
23c: prism
23d: emitting section
24: opening
25: air/water supply nozzle
26: light source unit
27: light source controller
28: light guide
29: connector
32: image pickup element
33: image pickup controller
34: CDS/AGC circuit
35: A/D converter
36: communication I/F
37: communication I/F
38: reception unit
39: signal processing unit
40: display controller
41: system controller
42: static image storage unit
58: irradiation region-recognizing section
59: learning model
60: pattern matching-processing section
60a: template image storage section
61: measurement marker setting section
62: marker table
80: intersection line
82: gradation
BF: blue color filter
GF: green color filter
RF: red color filter
BP: blue image
GP: green image
RP: red image
CR1, CR2: central region
M1, M2, M3: cruciform marker
Ma: measurement marker
tm1, tm2, tm3, tm4, tm5: tumor
SP: spot
SP1, SP2, SP3, SP4, SP5: spot
SR1, SR2: peripheral region
M4A, M4B, M4C, M5A, M5B, M5C: concentric circular marker
M6A, M6B, M6C: distorted concentric circular marker
P: polyp
WMB: wavelength range (of measurement marker)

## Claims

1. An endoscope system comprising:
an endoscope (12) that includes a beam light-emitting unit (23) irradiating a subject with measurement beam light and an image pickup optical system (21) provided in a distal end part (12d) at a position different from a position of the beam light-emitting unit and receiving light from the subject, the measurement beam light being emitted obliquely with respect to an optical axis of the image pickup optical system; and
a processor device (16),
wherein the processor device includes a processor that acquires a subject image that is obtained on the basis of the light received by the image pickup optical system and includes a beam irradiation region appearing on the subject due to irradiation with the measurement beam light, recognizes the beam irradiation region from the subject image, and causes a display to display a measurement image in which a measurement marker to be used to measure a size of the subject is displayed in the subject image on the basis of the beam irradiation region; **characterized in that**:
the beam irradiation region of the subject image has a pattern having a specific shape that includes a white central region and a peripheral region covering a periphery of the central region and having a feature quantity based on the measurement beam light irradiated on the subject, and
wherein the processor recognizes the beam irradiation region having the pattern having the specific shape; and wherein:
the feature quantity is at least one of a color of the measurement beam light, a color other than the color of the measurement beam light, or luminance, brightness, chroma saturation, or hue of the measurement beam light.

2. The endoscope system according to claim 1,
wherein the processor recognizes the beam irradiation region by a learning model that outputs the beam irradiation region in response to an input of the subject image.

3. The endoscope system according to claim 1,
wherein the processor recognizes the beam irradiation region by performing pattern matching processing on the subject image using a predetermined template image of the beam irradiation region.

4. The endoscope system according to any one of claims 1 to 3,
wherein the processor is capable of recognizing the beam irradiation region having a pattern of which the specific shape is deformed.

5. The endoscope system according to any one of claims 1 to 4,
wherein the specific shape is a circular shape.

6. The endoscope system according to any one of claims 1 to 4,
wherein the specific shape is a linear shape.

7. The endoscope system according to any one of claims 1 to 6,
wherein the processor receives the subject image including a plurality of color images, and
an amount of the measurement beam light is set to a specific amount of light and a pixel value of each of the color images is set to a maximum pixel value, so that a central region of the beam irradiation region is white.

8. The endoscope system according to any one of claims 1 to 7,
wherein the processor displays the measurement marker, which corresponds to a position of the beam irradiation region, in the measurement image.

9. The endoscope system according to any one of claims 1 to 7,
wherein the processor displays the measurement marker, which corresponds to a pattern of the beam irradiation region, in the measurement image.

10. A method of operating a processor of an endoscope system, the endoscope system including an endoscope that includes a beam light-emitting unit (23) configured to irradiate a subject with measurement beam light and an image pickup optical system (21) provided in a distal end part at a position different from a position of the beam light-emitting unit and configured to receive light from the subject, the measurement beam light being emitted obliquely with respect to an optical axis of the image pickup optical system, and a processor device (16) that includes the processor, the method comprising:
causing the processor to execute
a step of generating a subject image that is based on digital image signals output by the image pickup optical system and includes a beam irradiation region appearing on the subject due to irradiation with the measurement beam light,
a step of recognizing the beam irradiation region from the subject image, and
a step of causing a display to display a measurement image in which a measurement marker to be used to measure a size of the subject is displayed in the subject image on the basis of the beam irradiation region; **characterized in that**:
the beam irradiation region of the subject image has a pattern having a specific shape that includes a white central region and a peripheral region covering a periphery of the central region and having a feature quantity based on the measurement beam light irradiated on the subject, and
wherein the processor recognizes the beam irradiation region having the pattern having the specific shape;
wherein the feature quantity is at least one of a color of the measurement beam light, a color other than the color of the measurement beam light, or luminance, brightness, chroma saturation, or hue of the measurement beam light.

## Patentansprüche

1. Endoskopsystem, umfassend:
ein Endoskop (12), das eine Strahllicht-Emissionseineinheit (23), die ein Motiv mit Messstrahllicht bestrahlt, und ein optisches Bildaufnahmesystem (21), das in einem distalen Endteil (12d) an einer Position, die sich von einer Position der Strahllicht-Emissionseinheit unterscheidet, vorgesehen ist und Licht von dem Motiv empfängt, enthält, wobei das Messstrahllicht schräg in Bezug auf eine optische Achse des optischen Bildaufnahmesystems emittiert wird; und
eine Prozessorvorrichtung (16),
wobei die Prozessorvorrichtung einen Prozessor enthält, der ein Motivbild, das auf der Grundlage des Lichts, das durch das optische Bildaufnahmesystem empfangen wird, erhalten wird und einen Strahlbestrahlungsbereich, der aufgrund von Bestrahlung mit dem Messstrahllicht auf dem Motiv erscheint, enthält, erfasst, den Strahlbestrahlungsbereich aus dem Motivbild erkennt und eine Anzeige veranlasst, ein Messbild, in dem eine Messmarkierung, die verwendet werden soll, um eine Größe des Motivs zu messen, in dem Motivbild auf der Grundlage des Strahlbestrahlungsbereichs angezeigt wird, anzuzeigen; **dadurch gekennzeichnet, dass**:
der Strahlbestrahlungsbereich von dem Motivbild ein Muster mit einer spezifischen Form, die einen weißen Mittelbereich und einen peripheren Bereich, der eine Peripherie des Mittelbereichs abdeckt, enthält, und mit einer Merkmalsmenge, die auf dem auf das Motiv bestrahlten Messstrahllicht basiert, aufweist, und
wobei der Prozessor den Strahlbestrahlungsbereich mit dem Muster mit der spezifischen Form erkennt; und wobei:
die Merkmalsmenge mindestens eines von einer Farbe des Messstrahllichts, einer Farbe, die von der Farbe des Messstrahllichts verschieden ist, und Leuchtdichte, Helligkeit, Chroma-Sättigung oder Farbton des Messstrahllichts ist.

2. Endoskopsystem nach Anspruch 1,
wobei der Prozessor den Strahlbestrahlungsbereich durch ein Lernmodell, das den Strahlbestrahlungsbereich als Reaktion auf eine Eingabe des Motivbildes ausgibt, erkennt.

3. Endoskopsystem nach Anspruch 1,
wobei der Prozessor den Strahlbestrahlungsbereich durch Durchführen von Musterabgleichsverarbeitung an dem Motivbild unter Verwendung eines vorbestimmten Vorlagenbildes des Strahlbestrahlungsbereichs erkennt.

4. Endoskopsystem nach einem der Ansprüche 1 bis 3,
wobei der Prozessor in der Lage ist, den Strahlbestrahlungsbereich mit einem Muster, dessen spezifische Form verformt ist, zu erkennen.

5. Endoskopsystem nach einem der Ansprüche 1 bis 4,
wobei die spezifische Form eine Kreisform ist.

6. Endoskopsystem nach einem der Ansprüche 1 bis 4,
wobei die spezifische Form eine lineare Form ist.

7. Endoskopsystem nach einem der Ansprüche 1 bis 6,
wobei der Prozessor das Motivbild, das mehrere Farbbilder enthält, empfängt, und eine Menge des Messstrahllichts auf eine spezifische Lichtmenge eingestellt wird und ein Pixelwert jedes der Farbbilder auf einen Maximalpixelwert eingestellt wird, so dass ein Mittelbereich des Strahlbestrahlungsbereichs weiß ist.

8. Endoskopsystem nach einem der Ansprüche 1 bis 7,
wobei der Prozessor die Messmarkierung, die einer Position des Strahlbestrahlungsbereichs entspricht, in dem Messbild anzeigt.

9. Endoskopsystem nach einem der Ansprüche 1 bis 7,
wobei der Prozessor die Messmarkierung, die einem Muster des Strahlbestrahlungsbereichs entspricht, in dem Messbild anzeigt.

10. Verfahren des Betreibens eines Prozessors eines Endoskopsystems, wobei das Endoskopsystem ein Endoskop, das eine Strahllicht-Emissionseinheit (23), die so konfiguriert ist, dass sie ein Motiv mit Messstrahllicht bestrahlt, und ein optisches Bildaufnahmesystem (21), das in einem distalen Endteil an einer Position, die sich von einer Position der Strahllicht-Emissionseinheit unterscheidet, vorgesehen ist und so konfiguriert ist, dass es Licht von dem Motiv empfängt, enthält, wobei das Messstrahllicht schräg in Bezug auf eine optische Achse des optischen Bildaufnahmesystems emittiert wird, und eine Prozessorvorrichtung (16), die den Prozessor enthält, wobei das Verfahren umfasst:
Veranlassen, dass der Prozessor ausführt:
einen Schritt des Erzeugens eines Motivbildes, das auf digitalen Bildsignalen basiert, die von dem optischen Bildaufnahmesystem ausgegeben werden, und einen Strahlbestrahlungsbereich, der aufgrund von Bestrahlung mit dem Messstrahllicht auf dem Motiv erscheint, enthält,
einen Schritt des Erkennens des Strahlbestrahlungsbereichs aus dem Motivbild, und
einen Schritt des Veranlassens, dass eine Anzeige ein Messbild anzeigt, in dem eine Messmarkierung, die verwendet werden soll, um eine Größe des Motivs zu messen, in dem Motivbild auf der Grundlage des Strahlbestrahlungsbereichs angezeigt wird;
**dadurch gekennzeichnet, dass**:
der Strahlbestrahlungsbereich von dem Motivbild ein Muster mit einer spezifischen Form, die einen weißen Mittelbereich und einen peripheren Bereich, der eine Peripherie des Mittelbereichs abdeckt, enthält, und mit einer Merkmalsmenge, die auf dem auf das Motiv bestrahlten Messstrahllicht basiert, aufweist, und
wobei der Prozessor den Strahlbestrahlungsbereich mit dem Muster mit der spezifischen Form erkennt;
wobei die Merkmalsmenge mindestens eine von einer Farbe des Messstrahllichts, einer Farbe, die von der Farbe des Messstrahllichts verschieden ist, und Leuchtdichte, Helligkeit, Chroma-Sättigung oder Farbton des Messstrahllichts ist.

## Revendications

1. Système d'endoscope comprenant :
un endoscope (12) qui inclut une unité d'émission de lumière en faisceau (23) irradiant un sujet avec une lumière en faisceau de mesure et un système optique de prise d'images (21) disposé dans une partie d'extrémité distale (12d) à une position différente d'une position de l'unité d'émission de lumière en faisceau et recevant de lumière du sujet, la lumière en faisceau de mesure étant émise obliquement par rapport à un axe optique du système optique de prise d'images ; et
un dispositif processeur (16),
dans lequel le dispositif processeur inclut un processeur qui acquiert une image de sujet qui est obtenue sur la base de la lumière reçue par le système optique de prise d'images et inclut une région d'irradiation de faisceau apparaissant sur le sujet en raison de l'irradiation avec la lumière en faisceau de mesure, reconnaît la région d'irradiation de faisceau à partir de l'image de sujet, et amène un affichage à afficher une image de mesure dans laquelle un marqueur de mesure à utiliser pour mesurer une taille du sujet est affiché dans l'image de sujet sur la base de la région d'irradiation de faisceau ; **caractérisé en ce que** :
la région d'irradiation de faisceau de l'image sujet présente un motif ayant une forme spécifique qui inclut une région centrale blanche et une région périphérique recouvrant une périphérie de la région centrale et ayant une quantité de caractéristique basée sur la lumière en faisceau de mesure irradiée sur le sujet, et
dans lequel le processeur reconnaît la région d'irradiation de faisceau ayant le motif ayant la forme spécifique ; et dans lequel :
la quantité de caractéristique est au moins l'une d'une couleur de la lumière en faisceau de mesure, d'une couleur autre que la couleur de la lumière en faisceau de mesure, ou de luminance, de luminosité, de saturation de chroma ou de teinte de la lumière en faisceau de mesure.

2. Système d'endoscope selon la revendication 1,
dans lequel le processeur reconnaît la région d'irradiation de faisceau par un modèle d'apprentissage qui produit la région d'irradiation de faisceau en réponse à une entrée de l'image de sujet.

3. Système d'endoscope selon la revendication 1,
dans lequel le processeur reconnaît la région d'irradiation de faisceau en effectuant un traitement de correspondance de motifs sur l'image de sujet en utilisant une image modèle prédéterminée de la région d'irradiation de faisceau.

4. Système d'endoscope selon l'une quelconque des revendications 1 à 3,
dans lequel le processeur est capable de reconnaître la région d'irradiation de faisceau ayant un motif dont la forme spécifique est déformée.

5. Système d'endoscope selon l'une quelconque des revendications 1 à 4,
dans lequel la forme spécifique est une forme circulaire.

6. Système d'endoscope selon l'une quelconque des revendications 1 à 4,
dans lequel la forme spécifique est une forme linéaire.

7. Système d'endoscope selon l'une quelconque des revendications 1 à 6,
dans lequel le processeur reçoit l'image de sujet incluant une pluralité d'images en couleur, et
une quantité de la lumière en faisceau de mesure est réglée à une quantité spécifique de lumière et une valeur de pixel de chacune des images en couleur est réglée à une valeur de pixel maximale, de sorte qu'une région centrale de la région d'irradiation de faisceau soit blanche.

8. Système d'endoscope selon l'une quelconque des revendications 1 à 7,
dans lequel le processeur affiche le marqueur de mesure, qui correspond à une position de la région d'irradiation de faisceau, dans l'image de mesure.

9. Système d'endoscope selon l'une quelconque des revendications 1 à 7,
dans lequel le processeur affiche le marqueur de mesure, qui correspond à un motif de la région d'irradiation de faisceau, dans l'image de mesure.

10. Procédé de fonctionnement d'un processeur d'un système d'endoscope, le système d'endoscope incluant un endoscope qui inclut une unité d'émission de lumière en faisceau (23) configurée pour irradier un sujet avec une lumière en faisceau de mesure et un système optique de prise d'images (21) disposé dans une partie d'extrémité distale à une position différente d'une position de l'unité d'émission de lumière en faisceau et configuré pour recevoir de lumière du sujet, la lumière en faisceau de mesure étant émise obliquement par rapport à un axe optique du système optique de prise d'images, et un dispositif processeur (16) qui inclut le processeur, le procédé comprenant :
amener le processeur à exécuter
une étape consistant à générer une image de sujet qui est basée sur des signaux d'images numériques produits par le système optique de prise d'images et inclut une région d'irradiation de faisceau apparaissant sur le sujet en raison de l'irradiation avec la lumière en faisceau de mesure,
une étape consistant à reconnaître la région d'irradiation de faisceau à partir de l'image de sujet, et
une étape consistant à amener un affichage à afficher une image de mesure dans laquelle un marqueur de mesure à utiliser pour mesurer une taille du sujet est affiché dans l'image de sujet sur la base de la région d'irradiation de faisceau ; **caractérisé en ce que** :
la région d'irradiation de faisceau de l'image sujet présente un motif ayant une forme spécifique qui inclut une région centrale blanche et une région périphérique recouvrant une périphérie de la région centrale et ayant une quantité de caractéristique basée sur la lumière en faisceau de mesure irradiée sur le sujet, et
dans lequel le processeur reconnaît la région d'irradiation de faisceau ayant le motif ayant la forme spécifique ;
dans lequel la quantité de caractéristique est au moins l'une d'une couleur de la lumière en faisceau de mesure, d'une couleur autre que la couleur de la lumière en faisceau de mesure, ou de luminance, de luminosité, de saturation de chroma ou de teinte de la lumière en faisceau de mesure.
